# EUROPEAN PATENT APPLICATION

(11) **EP 0 549 263 A2**
(43) Date of publication of application: **30.06.1993**
(21) Application number: 92311538.0
(22) Date of filing: 17.12.1992
(51) Int. Cl.: C07C 213/02, C07C 217/84, C07D 317/66, C07D 491/04

(54) **A process for the preparation of N-alkyl-3,4-dialkyloxyanilines and derivatives thereof**

(30) Priority: 27.12.1991 IL 100537
(71) Applicant: CHEMAGIS LTD., Tel Aviv 61090 (IL)
(72) Inventor: Cherkez, Stephen, Ramat Gan (IL); Brand, Michael, Raanana (IL); Fikhte, Boris, Rehovot (IL)
(74) Representative: Tubby, David George

(57) **Abstract**

The invention provides a process for the preparation of N-alkyl-3,4-dialkyloxyanilines and derivatives thereof of the formula I 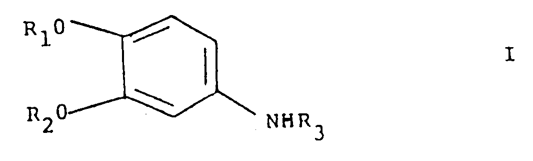
wherein R₁ and R₂ are each identical or different lower alkyl groups or R₁ and R₂ taken together form a methylene bridge and R₃ is H or lower alkyl comprising bromination of a compound of formula II 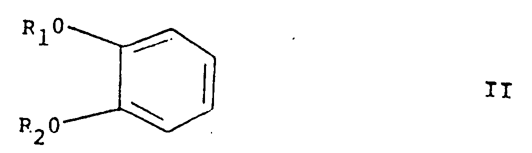
wherein R₁ and R₂ are as defined above to form a compound of formula III 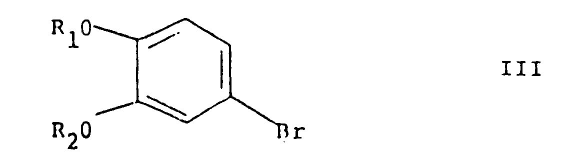
and then reacting said compound with an aqueous alkyl amine or ammonia of the formula R₃NH₂ wherein R₃ is as defined above to effect amination of the bromo amination of the bromo intermediate of formula III to form the compounds of formula I.

The present invention relates to a process for the preparation of N-alkyl-3,4-dialkyloxyanilines and derivatives thereof.

More particularly the present invention relates to a process for the preparation of N-alkyl-3,4-dialkyloxyanilines, which compounds can be used as intermediates in the preparation of quinazolinone and quinazolin- thione derivatives having antibacterial properties.

Thus the compounds produced by the process of the present invention can be used, e.g., to produce the known antibacterial compound oxolinic acid, which is 5-ethyl-5,8-dihydro-8-oxo-1,3-dioxolo[4,5-g] quinoline-7- carboxylic acid, and its salts.

In U.S. Patent 3,748,331 there is described inter alia, a process forthe preparation of compound of formula A 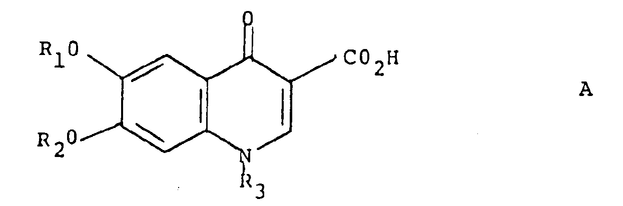
wherein R₃ signifies an alkyl radical of 1 to 5 carbon atoms and R₁=R₂=methylene bridge from compounds of formula I

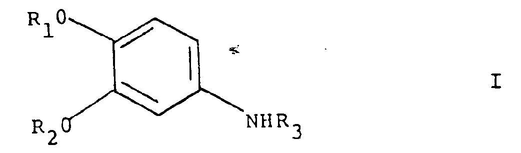

In said patent, compounds of formula I are prepared by a three-step synthesis (Scheme 1) involving:
a. nitration of 1,2-methylenedioxybenzene (MDB) (3a) to produce the nitro compound (4a) ;
b. subsequent reduction of 4a with hydrogen and a suitable catalyst (e.g. palladium on carbon) to produce 3,4-methylenedioxyaniline (MDA) (2b); and
c. alkylation of 2b to Produce N-alkyl MDA (I, R₃=alkyl).

The following scheme illustrates the known synthesis of N-alkyl MDA.

## Description

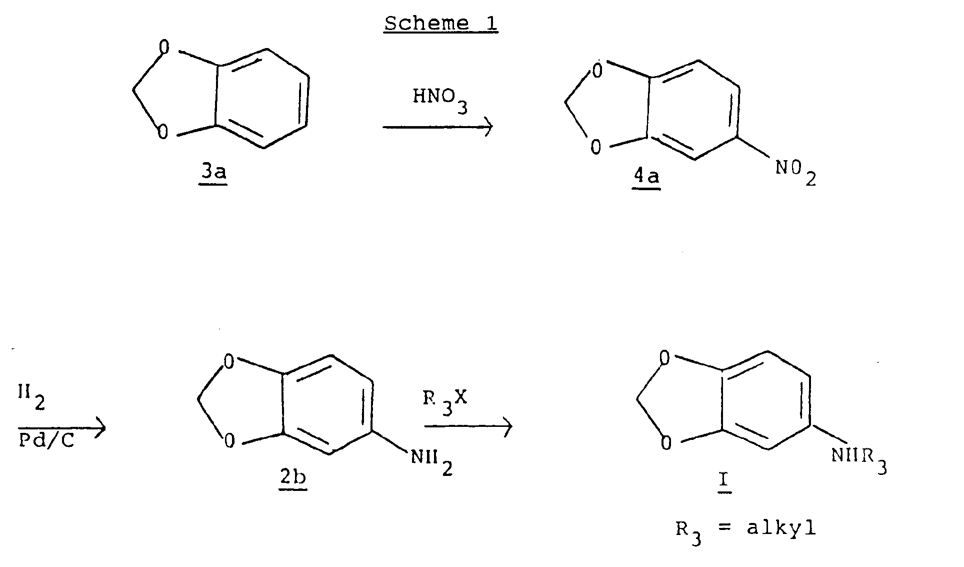

Products of formula A can be manufactured either directly from I (R₃=alkyl) or indirectly from compounds in which R₃=H by alkylation.

There are several disadvantages to the above synthesis. The nitration of3a involves large volumes of hazardous nitric and sulfuric acids which must be disposed of after the nitro compound 4a is filtered from the reaction mixture. Thus, a serious ecological problem arises in this synthesis. A separate filtration of the intermediate nitro compound 4a must be performed, a step which is not required in the novel process of the present invention as described hereinafter.

The reduction of 4a to 2b involves using expensive hydrogenation catalysts such as palladium or platinum which make this synthesis less attractive economically.

Again, in order to separate the catalyst from the reaction mixture, an additional filtration step is required. Finally, the alkylation of compounds of formula I wherein R₃ is H to give a product of formula I wherein R₃ is alkyl is also problematic in that dialkylation can occur, resulting in undesired by-products which must be removed from the desired product. In addition, some unreacted raw-material may remain and contaminate the desired product.

According to the present invention there is now provided a process for the preparation of N-alkyl-3,4-dialkyloxyanilines and derivatives thereof of the formula I 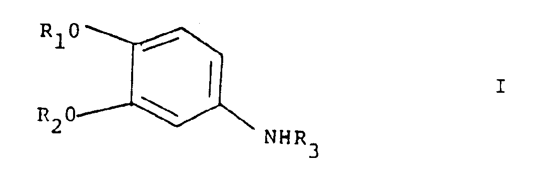
wherein R₁ and R₂ are each identical or different lower alkyl groups or R₁ and R₂ taken together form a methylene bridge and R₃ is H or lower alkyl comprising bromination of a compound of formula II 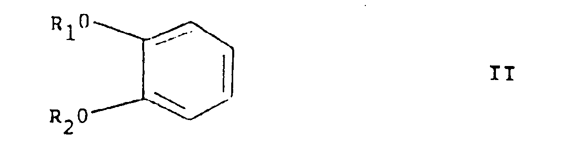 wherein R₁ and R₂ are as defined above to form a compound of formula III. 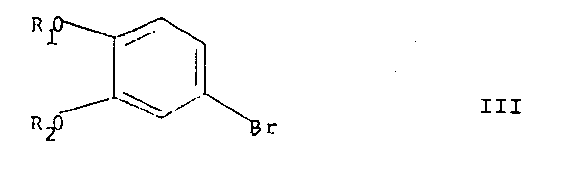
and then reacting said compound with an aqueous alkyl amine or ammonia with the formula R₃NH₂ wherein R₃ is as defined above to effect amination of the bromo intermediate of formula III to form the compounds of formula I.

As will be realized, in contradistinction to the above-described prior art, the process of the present invention involves only two synthetic steps:
a. bromination of a compound of formula II to afford the intermediate 4-bromo-1,2-dialkoxybenzene of formula III as defined; and
b. reaction of the compound of formula III with aqueous alkyl amine or ammonia to afford the product of formula I wherein R₃=H or alkyl in high yield.

The following scheme (Scheme 2) illustrates the general process of the present invention which can be used to prepare a variety of intermediates for quinoline derivatives and related compounds.

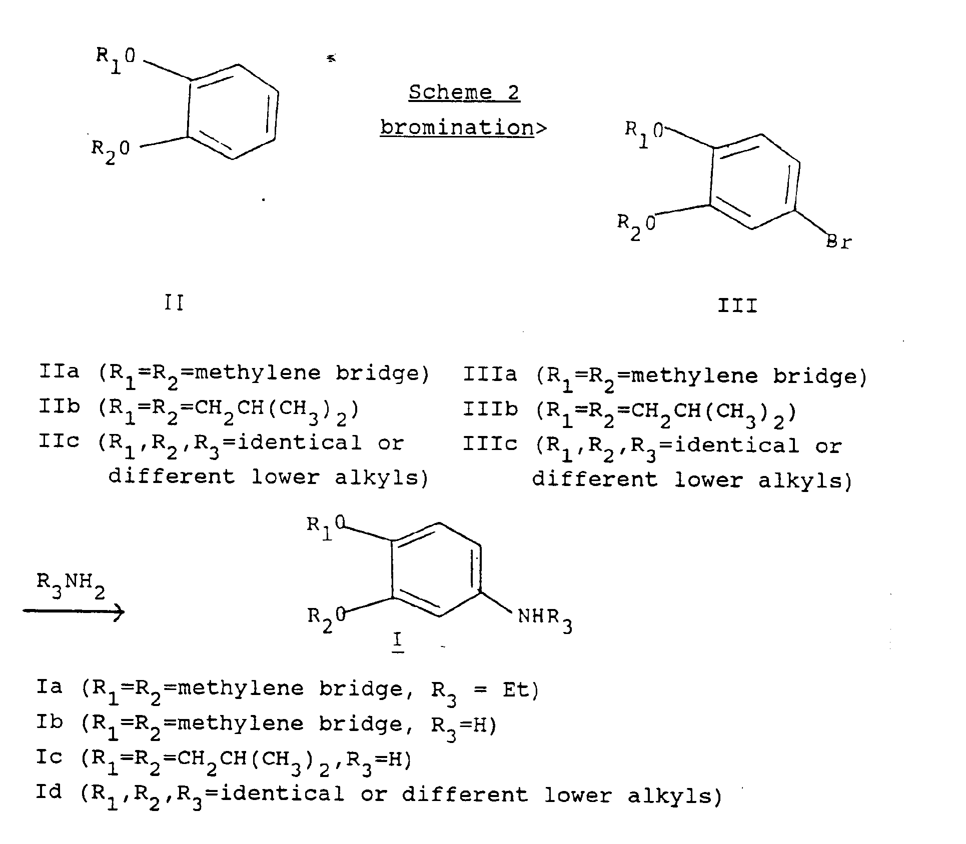

The bromination step is convenient and easy to perform. The bromine may be sourced from inexpensive reagents (e.g. aqueous hydrobromic acid and hydrogen peroxide). Reaction volumes are kept to a minimum as compared to the previous synthesis in which large volumes of nitric and sulfuric acids are required. The bromo intermediates of formula III are oils and need not be isolated by filtration as was the case with the nitro compound 4a. The reaction of the bromo intermediate of formula III with the cheap and readily available aqueous alkyl amine or ammonia afford the product of formula I practically free of disubstituted or unsubstituted impurities. The separation of I from the reaction medium involves a simple extraction using a convenient organic water-immiscible solvent such as toluene. Evaporation of the organic solvent provides the crude product I which can be purified either by high-vacuum distillation or by crystallization of its salts with suitable acids or can be used as such. The products obtained are suitable for preparing oxolinic acid, its derivatives and salts.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1

### Preparation of4-bromo-1,2-(methylenedioxy)benzene (Bromo MDB)

A flask equipped with stirrer, thermometer, and condenser is charged with 1,2-methylenedioxybenzene (30.0g), methanol (59g), glacial acetic acid (13.0g) and water (10g). The reaction mixture is stirred and 48% HBr (28.0ml) is added dropwise during 1 hour taking care that the temperature does not rise above 20°C. After the addition the mixture is heated to 35°C, and 32% H₂0₂ (28.9g) is added during 30 minutes, keeping the temperature in the range of45-50°C. After the addition, the mixture is stirred at 40-45°C for 2 hours. A sample is removed from the lower organic layer and the completion of the reaction is verified by GC. The reaction mixture is cooled to 25°C. The lower organic layer is separated and washed with a 10% solution of NaHS0₃ (50ml) to decompose the excess remaining H₂0₂. After treatment, the weight of the organic layer amounts to 52.0g and consists of bromo MDB having an assay of 85.5%. The crude bromo MDB is distilled under vacuum at 5mm pressure and at a temperature of 118-124°C to afford 44.5g of pure bromo MDB. This corresponds to a yield of 90% based on MDB.

### Example 2

### Preparation of N-ethyl-3,4-(methylenedioxy)aniline (N-ethyl MDA)

A 3-liter autoclave is charged with bromo MDB (333g) (assay 85%), 70% aqueous ethyl amine (446g), CuS0₄.5H₂0 (52g) and anh. K₂C0₃ (213g). The reaction mixture is heated to 110°-115°C and the mixture is allowed to react at this temperature for a period of 10 hours. The maximum pressure obtained during this period is 10-11 atm. Near the end of the heating period, the pressure falls to about 6 atm. A sample is removed for GC analysis. The GC trace indicates about 4% bromo-MDB remaining. The reaction mixture is cooled to 80°C. Water (500ml) and toluene (300mi) are added. The mixture is heated to 80°C and stirred at this temperature for 1 hour. The stirring is stopped and the layers are allowed to separate for 30 minutes. The lower aqueous layer (850ml) is separated and discarded. The volume of the upper organic toluene layer is 500ml. The toluene is distilled off. The remaining residue (300g) is fractionally distilled (pressure -1 mm Hg, temperature 125°-135°C) to provide 170g of pure N-ethyl MDA (assay >98%). The reaction yield is 74% based,on the starting bromo MDB.

### Example 3

### Preparation of 3,4-(Methylenedioxy)aniline (MDA)

A 3-liter autoclave is charged with bromo MDB (255g) (assay 85%), 28% aqueous ammonia (540g), CuS0₄.5H₂0 (68g) and K₂C0₃ (278g). The reaction mixture is heated to 195°C and the mixture is allowed to react at this temperature for a period of 14 hours. The maximum pressure obtained during this period is 70 atm. Near the end of the heating period the pressure falls to about 50 atm. The reaction mixture is cooled to 80°C. Water (650ml) and toluene (400ml) are added. The mixture is heated to 80°C and stirred at this temperature for 1 hour. The stirring is stopped and the layers are allowed to separate for 30 minutes. The lower aqueous layer is separated and discarded. The toluene is distilled off. The remaining residue (500g) is fractionally distilled (pressure -16mm, temperature 140°-145°C) to provide 103g of pure MDA (assay >98%). The reaction yield is 70% based on the starting bromo MDB.

### Example 4

### Preparation of 1-bromo-3,4-diisobutoxybenzene

A flask equipped with stirrer, thermometer, and condenser is charged with 1,2-diisobutoxybenzene (55.0g), methanol (59g), glacial acetic acid (13.0g) and water (10g). The reaction mixture is stirred and 48% HBr (28.0ml) is added dropwise during 1 hour taking care that the temperature does not rise above 20°C. After the addition the mixture is heated to 35°C. 32% H₂0₂ (28.9g) is added during 30 minutes keeping the temperature in the range 45-50°C. Afterthe addition the mixture is stirred at40-45°C for2 hours. A sample is removed from the lower organic layer and the completion of the reaction is verified by GC. The reaction mixture is cooled to 25°C. The lower organic layer is separated and this layer is washed with a 10% solution of NaHS0₃ (50ml) to decompose the excess H₂0₂ remaining. After treatment, the weight of the organic layer amounts to 78.0g and consists of 1-bromo-3,4-diisobutoxybenzene. The crude bromo derivative is distilled under vacuum to afford 66g of pure 1-bromo-3,4-diisobutoxybenzene. This corresponds to a yield of 90% based on diisobutoxybenzene.

### Example 5

### Preparation of 3,4-diisobutoxyaniline

A 3-liter autoclave is charged with 1-bromo-3,4-diisobutoxybenzene (382g) (assay >85%), 28% aqueous ammonia (540g), CuS0₄.5H₂0 (68g) and K₂C0₃ (278g). The reaction mixture is heated to 195°C and the mixture is allowed to react at this temperature for a period of 14 hours. The maxmium pressure obtained during this period is 70 atm. Near the end of the heating period the pressure falls to about 50 atm. The reaction mixture is cooled to 80°C. Water (650ml) and toluene (400ml) are added. The mixture is heated to 80°C and stirred at this temperature for 1 hour. The stirring is stopped and the layers are allowed to separate for 30 minutes. The lower aqueous layer is separated and discarded. The toluene is distilled off. The remaining residue is fractionally distilled to provide 179g of pure 3,4-diisobutoxyaniline (assay >98%). The reaction yield is 70% based on the starting 1-bromo-3,4-diisobutoxybenzene.

### Example 6

### Preparation of N-ethyl-3,4-diisobutoxyaniline

A 3-liter autoclave is charged with 1-bromo-3,4-diisobutoxybenzene (500g) (assay 85%), 70% aqueous ethyl amine (446g), CuS0₄.5H₂0 (52g) and K₂C0₃ (213g). The reaction mixture is heated to 110°-115°C and the mixture is allowed to react at this temperature for a period of 10 hours. The maximum pressure obtained during this period is 10-11 atm. Near the end of the heating period the pressure falls to about 6 atm. The reaction mixture is cooled to 80°C. Water (500ml) and toluene (300mi) are added. The mixture is heated to 80°C and stirred at this temperature for 1 hour. The stirring is stopped and the layers are allowed to separate for 30 minutes. The lower aqueous layer is separated and discarded. The toluene is distilled off. The remaining residue is fractionally distilled to provide 260g of pure N-ethyl-3,4-diisobutoxyaniline (assay >98%). The reaction yield is 70% based on the starting 1-bromo-3,4-diisobutoxybenzene.

### Example 7

### Preparation of Oxolinic acid and its Sodium Salt

### Stage 1: Preparation of Diethyl-N-ethyl methylene dioxyaniline methylene malonate

A 3-necked flask provided with stirrer, thermometer and condenser in downward distillation position is charged with N-ethyl MDA(33.0g) and diethylethoxymethylenemalonate (44.0g). The mixture is heated with stirring to 120°-125°C and is maintained at this temperature for 1.5 hours. During this period the ethanol produced in the reaction is collected. Vacuum (-100mmHg) is applied for about 30 minutes at 120°-125°C. The vacuum is released. The residue remaining in the reaction vessel consists of a dark brown viscous oil.

### Stage 2: Preparation of Crude Oxolinic Acid

To the stirred oily residue of diethyl-N-ethyl methylene dioxyaniline methylene malonate (67.0g) from Stage 1 at 80°-90°C is added acetic anhydride (165ml). The reaction mixture is stirred and conc. H₂S0₄ (73ml) is added dropwise at 90°C during 1 hour. An exothermic reaction results and the temperature rises to 120°C. At the end of the addition water (400ml) is added to the reaction mixture dropwise during 45 minutes. At the end of the addition, the mixture is heated at reflux for 16 hours. The resulting suspension is cooled to 25°C, filtered and the cake is washed with water until the water washings are no long acidic (about 100ml water is required). Finally the cake is washed with acetone and is dried at 80°C to afford 47g (90% yield) of crude oxolinic acid.

### Stage 3: Preparation of the Sodium Salt of Oxolinic Acid

A flask is charged with the crude oxolinic acid (47g), water (100mi), 50% NaOH solution (12ml) and DMF (100ml). The mixture is heated with stirring to 85°-90°C to obtain a solution. This solution is clarified by filtering the hot mixture. The clarified solution is cooled to 25°C and is stirred at this temperature for 2 hours. The resulting suspension is filtered. The cake is washed with DMF (2 x 25 ml) and acetone (2 x 25 ml). The product is dried at 50°C to afford 55.3g of sodium salt of oxolinic acid.

### Stage 4: Preparation of Oxolinic Acid from the Sodium Salt

A flask is charged with the sodium salt of oxolinic (55.3g). Water (50ml) and DMF (60ml) are added. The mixture is heated to 80°C to obtain a solution and to this hot solution is added 32% HCI (20ml) dropwise during 30 minutes. The mixture is cooled to 25°C and is stirred at this temperature for 1 hour. The suspension is filtered and the cake is washed with water (2 x 25 ml), DMF (2 x 25 ml) and acetone (2 x 25 ml). The product is dried at 80°C to afford 38g (73% yield) of oxolinic acid which conforms to specifications.

### Example 8

### Preparation of 1-ethyl-1,4-dihydro-6,7-diisobutoxy-4-oxo-3-quinoline carboxylic acid and its Sodium Salt

A 3-necked flask provided with stirrer, thermometer and condenser in downward distillation position is charged with N-ethyl-3,4-diisobutoxyaniline (53.0g) and diethyl ethoxymethylenemalonate (44.0g). The mixture is heated with stirring to 120°-125°C and is maintained at this temperature for 1.5 hours. During this period the ethanol produced in the reaction is collected. Vacuum (-100mm Hg) is applied for about 30 minutes at 120°-125°C. The vaccum is released. The residue remaining in the reaction vessel consists of a dark brown viscous oil.

To the stirred oily residue at 80°-90°C is added acetic anhydride (165mi). The reaction mixture is stirred and conc. H₂S0₄ (73ml) is added dropwise at 90°C during 1 hour. An exothermic reaction results and the temperature rises to 120°C. At the end of the addition water (400ml) is added to the reaction mixture dropwise during 45 minutes. At the end of the addition, the mixture is heated at reflux for 16 hours. The resulting suspension is cooled to 25°C, filtered and the cake is washed with water until the water washings are no longer acidic (about 100ml water is required). Finally the cake is washed with acetone and is dried at 80°C to afford 65g (90% yield) of 1-ethyl-1,4-dihydro-6,7-diisobutoxy-4-oxo-3-quinoline carboxylic acid.

A flask is charged with the crude 1b (65g), water (100ml), 50% HaOH solution (12ml) and DMF (100ml). The mixture is heated with stirring to 85°-90°C to obtain a solution. This solution is clarified by filtering the hot mixture. The clarified solution is cooled to 25°C and is stirred at this temperature for 2 hours. The resulting suspension is filtered. The cake is washed with DMF (2 x 25 ml) and acetone (2 x 25 ml). The product is dried at 50°C to afford 75g of sodium salt of (1b).

A flask is charged with the sodium salt of 1b (75g). Water (50ml) and DMF (60ml) are added. The mixture is heated to 80°C to obtain a solution and to this hot solution is added 32% HCI (20ml) dropwise during 30 minutes. The mixture is cooled to 25°C and is stirred at this temperature for 1 hour. The suspension is filtered and the cake is washed with water (2 x 25 ml), DMF (2 x 25 ml) and acetone (2 x 25 ml). The product is dried at 80°C to afford 52g (73% yield) of 1b which conforms to specifications.

### Example 9

### Preparation of 4-Hydroxy-6,7-diisobutoxy-3-quinoline carboxylic acid and its Sodium Salt

A 3-necked flask provided with stirrer, thermometer and condenser in downward distillation position is charged with 3,4-diisobutoxyaniline (47.4g) and diethylethoxymethylenemalonate (44.0g). The mixture is heated with stirring to 120°-125°C and is maintained at this temperature for 1.5 hours. During this period the ethanol produced in the reaction is collected. Vacuum ( 100mm Hg) is applied for about 30 minutes at 120°-125°C. The vacuum is released. The residue remaining in the reaction vessel consists of a dark brown viscous oil.

To the stirred oily residue at 80-90°C is added acetic anhydride (165mi). The reaction mixture is stirred and conc. H₂S0₄ (73ml) is added dropwise at 90°C during 1 hour. An exothermic reaction results and the temperature rises to 120°C. At the end of the addition water (400ml) is added to the reaction mixture dropwise during 45 minutes. At the end of the addition, the mixture is heated at reflux for 16 hours. The resulting suspension is cooled to 25°C, filtered and the cake is washed with water until the water washings are no longer acidic (about 100ml water is required). Finally the cake is washed with acetone and is dried at 80°C to afford 60g (90% yield) of 4-hydroxy-6,7-diisobutoxy-3-quinoline carboxylic acid (1c).

A flask is charged with the crude 1c (60g), water (100ml), 50% NaOH solution (12ml) and DMF (100mi). The mixture is heated with stirring to 80°-90°C to obtain a solution. This solution is clarified by filtering the hot mixture. The clarified solution is cooled to 25°C and is stirred at this temperature for 2 hours. The resulting suspension is filtered. The cake is washed with DMF (2 x 25ml) and acetone (2 x 25ml). The product is dried at 50oC to afford 70g of sodium salt of 1c.

A flask is charged with the sodium salt of 1c (75g). Water (50ml) and DMF (60 ml) are added. The mixture is heated to 80° to obtain a solution and to this hot solution is added 32% HCI (20ml) dropwise during 30 minutes. The mixture is cooled to 25°C and is stirred at this temperature for 1 hour. The suspension is filtered and the cake is washed with water ( 2 x 25ml), DMF (2 x 25ml) and acetone (2 x 25ml). The product is dried at 80°C to afford 48g (73% yield) of 1c which conforms to specifications.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A process for the preparation of N-alkyl-3,4-dialkyloxyanilines and derivatives thereof of the formula I 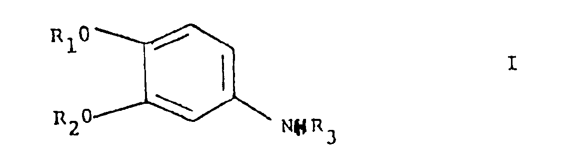
wherein R₁ and R₂ are each identical or different lower alkyl groups or R₁ and R₂ taken together form a methylene bridge, and R₃ is H or lower alkyl, comprising bromination of a compound of formula II 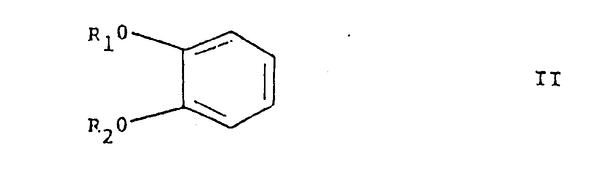
wherein R₁ and R₂ are as defined above to form a compound of formula III 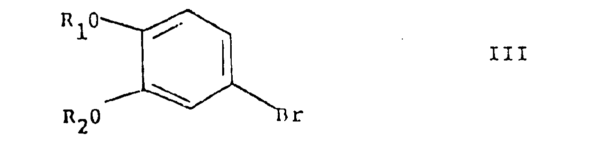
and then reacting said compound with an aqueous alkyl amine or ammonia of the formula R₃NH₂ wherein R₃ is as defined above to effect amination of the bromo intermediate of formula III to form the compounds of formula I.

2. A process according to claim 1 for the preparation of a compound of formula I wherein R₁ and R₂ taken together form a methylene bridge and R₃ is ethyl.

3. A process according to claim 1 for the preparation of a compound of formula I wherein R₁ and R₂ taken together form a methylene bridge and R₃ is hydrogen.

4. N-ethyl-3,4-(methylenedioxy)aniline whenever produced by the process of claim 1.

5. 3,4-(methylenedioxy)aniline whenever produced by the process of claim 1.

6. 3,4-diisobutoxyaniline whenever produced by the process of claim 1.

7. N-ethyl-3,4-diisobutoxyaniline whenever produced by the process of claim 1.

8. A process for the preparation of oxolinic acid and salts thereof comprising bromination of a compound of formula II 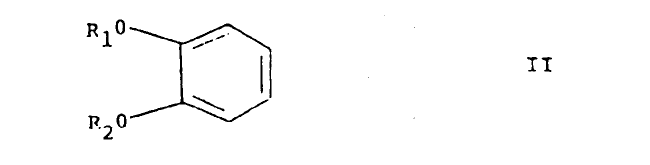
wherein R₁ and R₂ are methylene to form a compound of formula III, 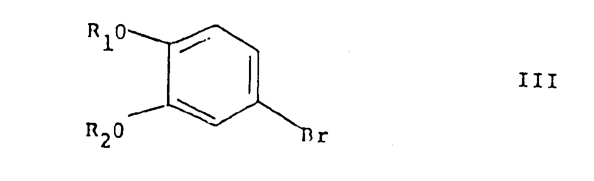
reacting said compound with an aqueous alkyl amine or ammonia of the formula R₃NH₂ wherein R₃ is hydrogen orethyl to form 3,4-(methylenedioxy)aniline or N-ethyl-3,4 (methylenedioxy)aniline respectively, and then preparing oxolinic acid or a salt thereof from either MDAor N-ethyl MDA by methods known per se and as described in example 7.

9. A process for the preparation of 1-ethyl-1,4-dihydro-6,7-diisobutoxy-4-oxo-3-quinoline carboxylic acid and salts thereof comprising bromination of a compound of formula II 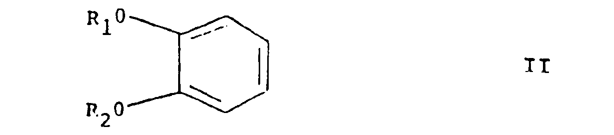
wherein R₁ and R₂ are each isobutoxy to form a compound of formula III, 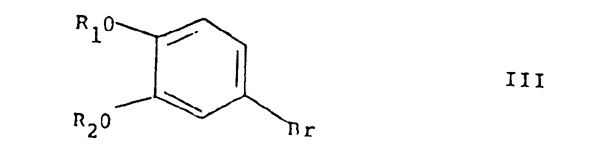
reacting said compound with an aqueous alkyl amine of the formula R₃NH₂ wherein R₃ is ethyl to form N-ethyl-3,4-diisobutoxyaniline and then preparing 1-ethyl-1,4-dihydro-6,7-diisobutoxy-4-oxo-3-quinoline carboxylic acid and salts thereof from N-ethyl-3,4- diisobutoxyaniline by methods known per se and as described in example 8.

10. A process for the preparation of 1,4-dihydro-6,7-diisobutoxy-4-oxo-3-quinoline carboxylic acid and salts thereof comprising bromination of a compound of formula II 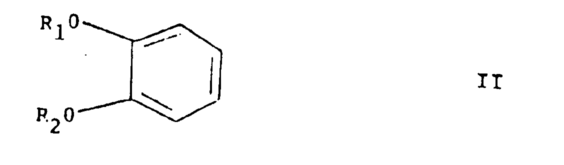
wherein R₁ and R₂ are each isobutoxy to form a compound of formula III, 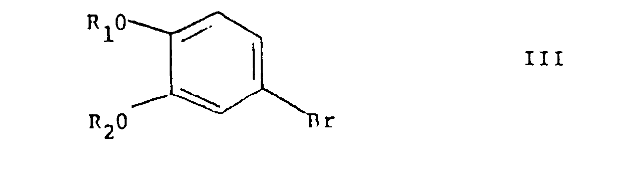
reacting said compound with ammonia to form 3,4-diisobutoxyaniline and then preparing 1,4-dihydro-6,7-diisobutoxy-4-oxo-3-quinoline carboxylic acid and salts thereof from 3,4- diisobutoxyaniline by methods known per se.
